# EUROPEAN PATENT SPECIFICATION

(11) **EP 3 023 074 B1**
(45) Date of publication and mention of the grant of the patent: **04.03.2020**
(21) Application number: 15195809.7
(22) Date of filing: 23.11.2015
(51) Int. Cl.: A61B 18/20, A61B 17/00, A61B 18/00

(54) **LASER SYSTEM FOR SKIN TREATMENT**
LASERSYSTEM ZUR HAUTBEHANDLUNG
SYSTÈME LASER POUR LE TRAITEMENT DE LA PEAU

(30) Priority: 22.11.2014 US 201462083178 P
(43) Date of publication of application: 25.05.2016
(73) Proprietor: Candela Corporation, Wayland, MA 01778 (US)
(72) Inventor: Shang, Xiaoming, Lexington, MA 02421 (US); Qiu, Jinze, Natick, MA 01760 (US); Jones, Christopher, Leicester, MA 01524 (US); Hsia, James, Weston, MA 02493 (US); Bhawalkar, Jayant, Auburndale, MA 02466 (US)
(74) Representative: Nederlandsch Octrooibureau

(56) References cited:
- US-A1- 2005 177 145
- US-A1- 2007 133 627
- US-A1- 2009 227 995
- US-A1- 2009 304 033

## Description

### TECHNOLOGY FIELD

The present apparatus and method relate generally to lasers, and more particularly to solid state lasers suitable for skin treatment.

### BACKGROUND

Lasers and other light-based devices are well established for treatment of several skin disorders as well as for aesthetic applications. Among the most widely used treatments are hair removal, removal of pigmented lesions, treatment of vascular lesions, and tattoo removal. Vascular lesions can be treated with light in the 500-1100nm range with an optimal wavelength range of 500-600nm. Most laser treatments of vascular lesions are using either a pulsed dye laser at 585-595nm or a frequency-doubled Nd:YAG laser at 532nm. The typical pulse durations for these treatments are between 0.1ms and 100ms. Pulsed dye lasers, while used widely for this application, have problems with limited life of the dye. The solid-state frequency-doubled Nd:YAG laser is an attractive alternative. However, it is difficult to achieve high energy millisecond regime pulse durations in a frequency doubled laser.

Benign vascular lesions of the skin are quite common. These are generally caused by an abnormal dilation of the dermal micro-vasculature. These range from port wine stains (PWS), a birth mark afflicting some 0.3% of newborns, to telangiectasia, commonly referred to as broken blood vessels which generally occur with age in sun exposed areas of the skin. Patients seek treatment because of the blemish these lesions cause in their appearance. PWS can be quite disfiguring, as they generally darken with age into deep purple colored patches. Often the lesions if not treated thicken and become raised which make the lesions even more prominent, disfiguring and more difficult to treat.

Pigmented lesions can also be treated with light in the 500-1100nm range. Pigmented lesions can simplistically be thought of as an overabundance of melanosomes in the epidermis, dermis, or both the epidermis and dermis although some lesions may also have a dermal vascular component. For pigmented lesion applications, q-switched pulses with pulse durations in the 5ns to 200ns range are desirable to better match the thermal relaxation time of the melanosomes. Q-switched lasers like alexandrite, ruby, Nd:YAG, or frequency-doubled Nd:YAG are used for treating epidermal and dermal pigmented lesions. Optical damage to melanosomes is more favorable for shorter wavelength in a range of 500-600nm. Because pigmented lesions and vascular lesions are often seen together, there is commercial motivation for combined treatment of the two conditions effectively.

Vascular lesions are most effectively and safely treated with pulse lasers. Typically, the vascular lesions in the skin are treated by pulsed laser radiation with wavelength where there is strong preferential blood absorption and pulse durations shorter or about equal to the thermal relaxation time of the blood vessels to be targeted.

Currently for the treatment of vascular lesions there are two types of lasers in common clinical use that satisfy these criteria. These are the flash lamp pumped pulse dye laser (PDL) and the frequency doubled Nd:YAG laser. Both lasers are used routinely today to treat small caliber telangiectatic vessels while the PDL tend to be favored for PWS. Due to their superior ability to selectively injure the dermal vasculature with minimal injury to the skin, they can be safely used to treat infants less than a year old without causing a scar.

The PDL laser is relatively expensive and requires periodic replacement of the dye due to photo degradation of the dye as the laser is used. The frequency doubled Nd:YAG laser used to treat vascular lesions is commonly referred to as the KTP laser. KTP or potassium titanyl phosphate (KTiOPO₄) is the non-linear crystal used to frequency double the 1.064 micron output of the Nd:YAG laser to generate its 532 nm output. The KTP lasers are expensive and therefore available to relatively few of the dermatologists who specialize in treating vascular lesions.

US patents numbers No. 6,547,781, 6554,825, 7,118,562, 7,639,721, 7,769,059 and 7,771,417 disclose different laser constructions and methods of skin treatment.

US Patent Application Publication 2009/0227995 to the same applicant and at least one common inventor describes a laser system for treating skin. The system includes a first solid-state laser for producing a first output beam, a second solid state laser for producing a second output beam, and a delivery device for directing the second output beam to a target region of skin. The second solid state laser is adapted to receive a first part of the first output beam and generate excitation in a rare-earth doped gain medium to produce the second output beam. The second output beam is for treating the skin.

### DEFINITIONS

Skin phototype (SPT) is a classification system based on a person's sensitivity to sunlight. The sensitivity to sunlight is measured using Fitzpatrick phototyping scale that classifies the response of different types of skin to sunlight using a value from 1 for light skin very sensitive to sunlight to a value of 6 for dark skin not very sensitive to sunlight.

Thermal lensing is a lensing effect induced by temperature gradients along and across the lasing or gain medium, for example Nd:YAG or Nd:YLF. The gain medium is usually hotter on the beam axis, compared with the outer regions, typically causing some transverse gradient of the refractive index.

KTP is abbreviation for Potassium Titanyl Phosphate, which is a crystal that exhibits nonlinear polarization properties and is widely used for laser light frequency doubling, also known as second harmonic generation

KDP is abbreviation for Potassium Dihydrogen Phosphate, which is a crystal that exhibits nonlinear polarization properties and is widely used for laser light frequency doubling, also known as second harmonic generation.

Nd:YAG is abbreviation for neodymium-doped yttrium aluminum garnet (Nd:Y₃Al₅O₁₂), a laser crystal that typically emits light at 1064nm.

Nd:YLF is abbreviation for neodymium-doped yttrium lithium fluoride (Nd: LiYF₄), a laser crystal that typically emits light at 1048nm.

Cr₄+:YAG is abbreviation for chromium-doped yttrium aluminum garnet, a crystal used for passive q-switching.

LBO is an abbreviation for lithium triborate (LiB₃O₅) a nonlinear optical crystal that can be used for frequency doubling.

### SUMMARY

The invention is defined in claims 1 and 13.

Disclosed is a treatment method for a segment of skin, the segment of the skin includes pigmented lesions and vascular lesions. The treatment is performed by application to the segment of skin of light with a wavelength between 500nm and 600nm and in particular with a wavelength of 524nm. The light is delivered to the treated segment of skin as a train of sub-pulses filling in an envelope pulse referred to as the macro pulse. In some examples the light is delivered to the treated segment of skin in a train of pulses as a combination of two wavelengths of 524nm 1048nm. In one example, both wavelengths are delivered in a sequence where one of the wavelengths could be delivered first with the second wavelength following the delivery of the first wavelength. In another example, both wavelengths could be delivered simultaneously to the segment of skin. The sub-pulses have a repetition rate of 10kHz - 1MHz and the macro pulses have a repetition rate of 1 to 10 Hz.

Described is also an apparatus configured to generate the train of pulses. The apparatus includes a free-running alexandrite laser configured to pump an Neodymium doped (Nd-doped) laser rod; a passive Q-switch and a second harmonic generator.

In one aspect of the method, the train of sub-pulses filling-in each macro pulse are in a range of 5ns to 200ns and the duration of the macro pulses is in a range 0.1ms to 100 ms. This may be achieved using a device for delivering the laser light to the treated segment of skin as a combination of two wavelengths of 524nm 1048nm and wherein fluence ratio of the 524nm wavelength to the 1048nm wavelength is between 1: 3 and 1 : 10.

In another aspect, the method of selecting the laser light wavelength delivered to treated segment of skin is by optimizing the laser light wavelength to absorption coefficient of blood (mixture of oxyhemoglobin and deoxyhemoglobin) and pigment (melanosomes). The delivery of the laser light to the treated segment of skin may take place in a train of sub-pulses with wavelength of 524nm.

Alternatively, delivering the laser light to the treated segment of skin may take place in a train of sub-pulses with wavelength of 532nm.

In another alternative, delivering the laser light to the treated segment of skin may take place in a train of sub-pulses including a combination of two wavelengths of 524nm and 1048nm. The sub-pulses may have a repetition rate of 10kHz - 1MHz. An envelope of a sequence of macro pulses of laser light may also have a repetition rate of 1Hz to 10 Hz.

In one embodiment, the method may further comprise using a combination of 1064nm and 532nm wavelengths for treating pigmented lesions, vascular lesions, and for skin toning, and delivering each of the wavelengths as a pulse train. The pulse train may be defined as each sub-pulse having a duration of 5ns to 200ns, and the duration of a macro pulse being 0.1ms to 100ms.

The method may also be carried out using the apparatus to deliver a combination of 1064nm and 532nm wavelengths for treating pigmented lesions, vascular lesions, and for skin toning, the energy being delivered as a pulse train, the pulse train defined as each sub-pulse having a duration of 5ns to200ns, the total duration of the pulse train being 0.1ms to 100ms.

In a particular embodiment, delivery is of a combination of 1064nm and 532nm wavelengths in a pulse train and the combination of 1064nm and 532nm is delivered at least as one of either a group of sequential delivery of each of the wavelengths or simultaneous delivery of both of the wavelengths.

The method is preferably performed using an alexandrite laser to pump a neodymium doped laser medium to produce the laser light. The alexandrite laser may also be configured to treat other dermatologic conditions.

### BRIEF LIST OF DRAWINGS AND THEIR DESCRIPTION

FIG. 1 is a graph illustrating the absorption coefficient spectra for oxyhemoglobin, deoxyhemoglobin and a melanosome from 300nm to 1000nm;
FIG. 2 is a graph illustrating the absorption coefficient spectra for oxyhemoglobin and deoxyhemoglobin from 500nm to 600nm;
FIG. 3 is a graph illustrating the absorption coefficient spectra for a 50:50 mixture of oxyhemoglobin and deoxyhemoglobin typically found in the superficial skin vasculature from 500nm to 600nm;
FIG. 4 is a graph illustrating 1/e penetration depth spectra (the inverse of the absorption coefficient) for a 50:50 mixture of oxyhemoglobin and deoxyhemoglobin typically found in the superficial skin vasculature from 500nm to 600nm;
FIG. 5 is an example of an apparatus for treatment of the combination of pigmented and vascular lesions;
FIG. 6 is a plot the amplitude versus time of an exemplary laser pulse train prescribed herein for the treatment of vascular and pigmented lesions on a skin segment; and
Fig 7 is another example of an apparatus for treatment of pigmented and vascular lesions on a skin segment where the Nd:YLF laser and the frequency doubling crystal resides in a detachable handpiece.

### DETAILED DESCRIPTION

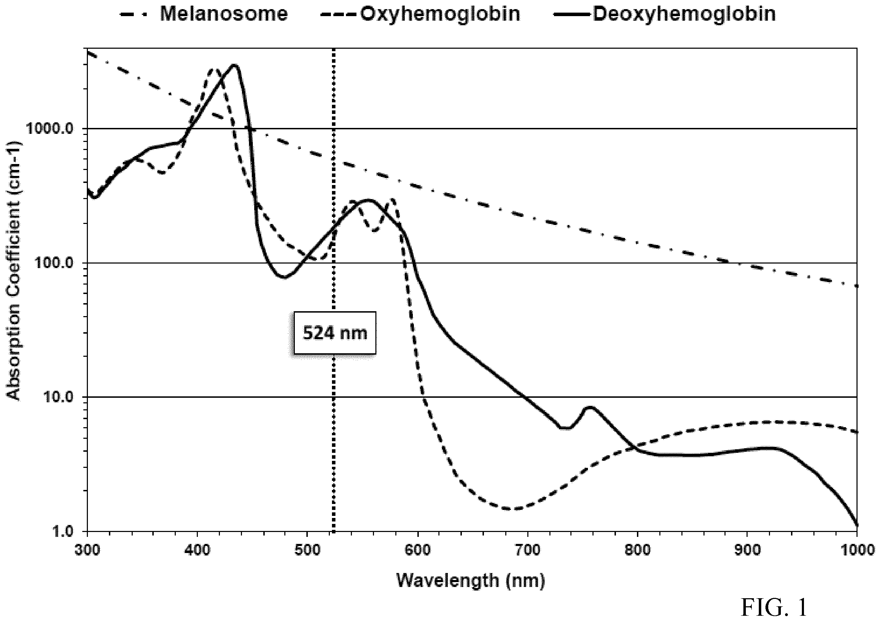

Pigmented lesions and vascular lesions are often seen together and because of this there is commercial motivation for a single treatment capable treating both of these dermatologic conditions effectively and efficiently. This can be done by optimizing the wavelength of light to the absorption coefficient of blood (mixture of oxyhemoglobin and deoxyhemoglobin) and pigment (melanosomes). FIG. 1 is a graph illustrating the absorption coefficient spectra for oxyhemoglobin, deoxyhemoglobin and a melanosome from 300nm to 1000nm. The 524nm laser wavelength is also shown for reference. This disclosure describes a method that allows effective combined treatment of the two dermatologic conditions with a carefully selected wavelength in the range of 500-600nm, and an apparatus for such treatment.

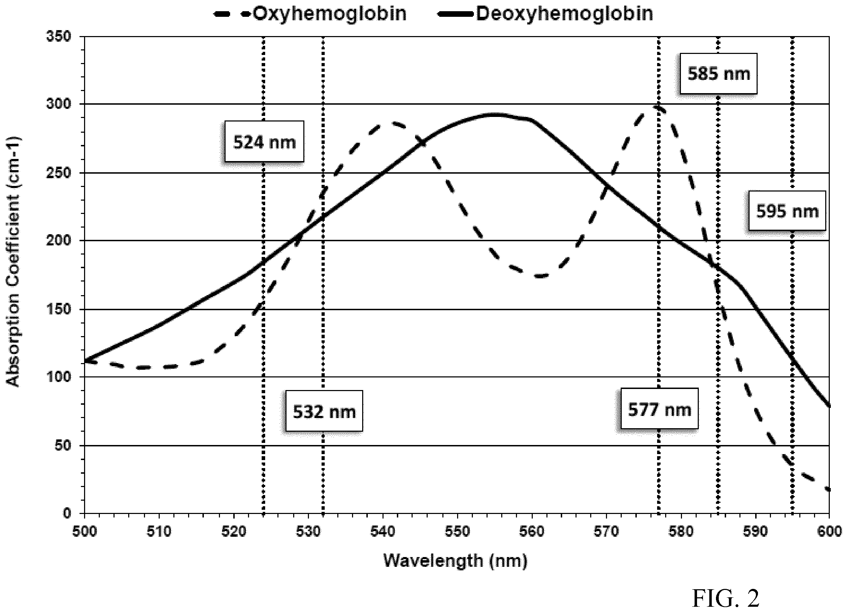

It is known that blood absorption (FIG. 2) at the wavelength of 524nm is similar to that at the wavelength of 585nm, higher than the wavelength of 595nm, but lower than at the wavelength of 532nm or 577nm. Previous work using pulsed dye lasers has shown that for vessels greater than 50 microns in diameter, light at 577nm does not have enough penetration into the vessels to coagulate them uniformly, and that light at 585nm, where the blood absorption is lower, is more effective for treating vessels with diameters in the range of 50 to 150 microns. Blood in the superficial dermal vessels is typically a 50:50 mixture of oxyhemoglobin and deoxyhemoglobin.

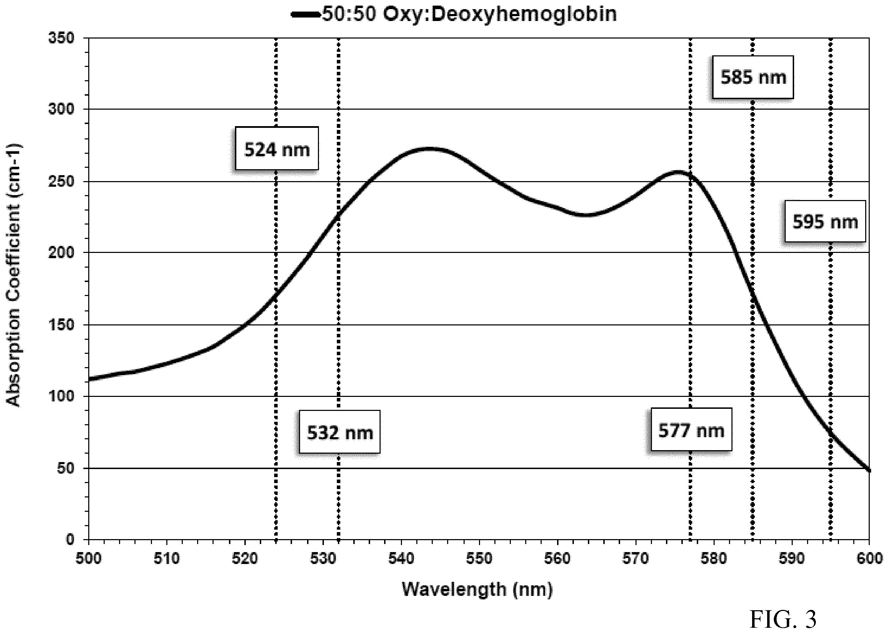

FIG. 3 is a graph illustrating the absorption coefficient spectra for a 50:50 mixture of oxyhemoglobin and deoxyhemoglobin typically found in the superficial skin vasculature from 500nm to 600nm. The figure clearly illustrates that the absorption coefficient for a 50:50 mixture of oxyhemoglobin and deoxyhemoglobin at a wavelength of 524nm is similar to the absorption at a wavelength 585nm.

Vascular lesions are most effectively and safely treated with pulse lasers and in particular with wavelength where there is strong preferential blood absorption of the laser light and laser light pulse durations are shorter or about equal to the thermal relaxation time of the blood vessels to be targeted. The authors of the current disclosure have found that using a wavelength between 500nm and 600nm with a pulse duration of 0.1 to 100ms closely meets this condition.

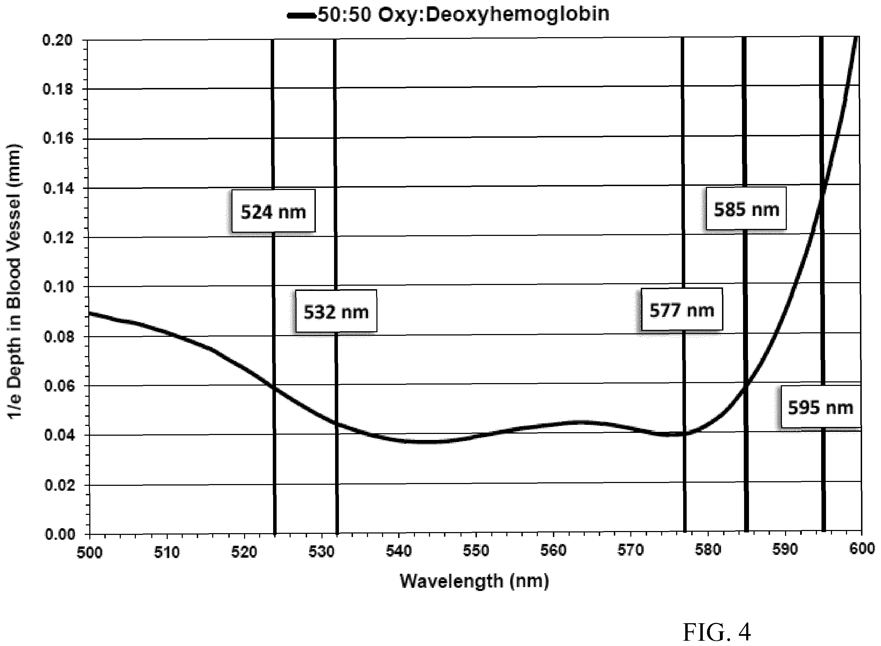

In order to achieve effective treatment of blood vessels, as a general rule of thumb, the penetration of light should be at least one half the vessel diameter. The penetration of light in the skin can be described by an exponential process. The 1/e penetration depth of the laser light in blood can be calculated as the inverse of the absorption coefficient (FIG. 4). The wavelength of 524nm is therefore expected to be more suitable for treating vessels with diameter of up to 135 micron, while 532nm is good for treating vessels up to 100 microns in diameter in the same way that 595nm is more effective than 577nm. The duration of the pulse is also important and depends on the diameters of the vessel. If the pulse duration is too long then energy is diffusing away from the target during the laser pulse reducing the resultant blood vessel temperature. Delivering the laser energy in short pulse widths allows more effective treatment as the energy is confined to the vessel. Port wine stains (PWS) vessels typically range in diameter from 10 to 300 microns, and can lie at depths 100 microns to 1mm from the skin surface. Larger diameter vessels beyond the typical diameter also could be treated, however higher laser fluences and longer pulse widths could be required leading to more damage to adjacent tissues.

**Table 1 below provides theoretically determined penetration into the skin of laser light of different wavelength.**

| Wavelength (nm) | 1/e Penetration* Depth (µm) | Typical Vessel Diameter (µm) | Typical Pulse Width (ms) |
|---|---|---|---|
| 524 | 60 | 40 - 135 | 0.8 - 10 |
| 532 | 45 | 30 - 100 | 0.5 - 5 |
| 577 | 40 | 30 - 90 | 0.5 - 4 |
| 585 | 60 | 40 - 135 | 0.8 - 10 |
| 595 | 135 | 90 - 305 | 4 - 50 |

| | | | |
|---|---|---|---|
| *For a 50:50 oxyhemoglobin:deoxyhemoglobin mixture. Note, larger vessel diameters can be effectively treated if compressed by the applicator. | | | |

In addition to treating vascular lesions, the 524nm wavelength is a suitable wavelength for treating pigmented lesions. As it is illustrated in FIG. 1, the 524nm wavelength is even more strongly absorbed by pigment (melanin in melanosomes) than the 755nm wavelength provided by the Q-switched alexandrite laser that is commonly used for treating pigmented lesions.

Pigmented lesions are effectively and safely treated with pulse lasers and in particular with wavelength where there is strong preferential absorption of the laser light by melanin and laser light pulse durations are shorter or about equal to the thermal relaxation time of the melanosomes to be targeted. Melanosomes are elliptical in shape having a major and minor diameter of 10 micron and 0.50 micron. Because of their small sizes, pulse widths less than 100ns are optimal for thermal confinement for melanosomes. The authors of the current disclosure used a wavelength between 500nm and 600nm with pulse duration of 5-500ns for treatment of pigmented lesions. The authors of the disclosure have experimentally determined that shorter laser light pulses produced higher incidences of hypopigmentation, while longer individual laser light pulses exceeded the time of the thermal relaxation of the melanosomes and were less effective in treating pigmented lesions.

When treating lesions that have both a vascular and pigmented component, the laser light pulses are applied to a segment of skin with pigmented lesions and vascular lesions with a laser light wavelength between 500nm and 600nm. The authors of the current disclosure have used a pulse train with individual sub-pulses in the train having an individual sub-pulse duration of 5ns to 500ns for treatment of melanosomes. The length of the macro or pulse train envelop could be selected to match the thermal relaxation time of the blood vessels and it could be 0.1miliseconds to 100 milliseconds. The combination of a long or macro pulse made-up of a train of multiple short sub-pulses within the long macro pulse train has proven to be more effective in treating a segment of skin with a combination of pigmented and vascular lesions to remove the lesions. The fluence range of the treatment of the combination of pigmented and vascular lesions by laser light with wavelength of 500nm to 600nm, could be from 3J/cm² to 30J/cm² depending on subject skin phototype, laser spot diameter, and duration of the pulse train.

Sagging skin is generally treated with light in the 500nm-1100nm range. For sagging skin treatment applications, both Q-switched pulses with pulse durations in the 5ns to 200ns range and longer pulses in the range of 0.1-100ms are used. Controlled heating of dermal collagen does not cause instant death of collagen producing cells (fibroblasts), but causes thermal activation of fibroblasts increasing new collagen deposition and thereby tightens sagging skin (skin rejuvenation). Q-switched infrared laser like Nd:YAG is frequently used to treat pigmented lesion and sagging skin. This type of treatment is often referred to as skin laser toning.

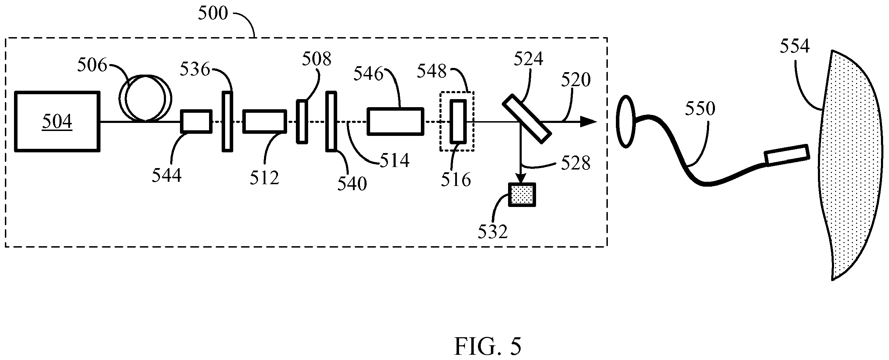

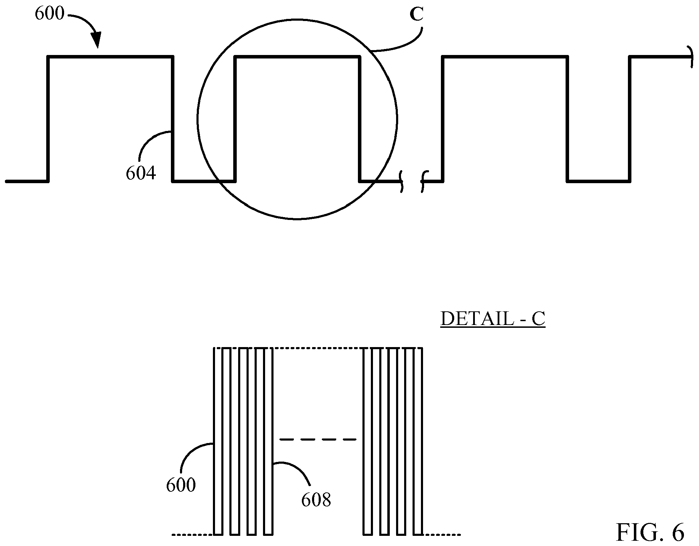

FIG. 5 is an example of an apparatus that supports combined treatment of the combination of pigmented and vascular lesions. Apparatus 500 supports the generation of a train of laser light pulses with 1ns to 200ns duration each within an envelope of 0.1ms to 100ms (FIG. 6). The envelope of the train of laser light pulses (macro pulses) could have a repetition rate of between 1Hz to 10 Hz. Such pulse trains obviate the need for a laser capable of generating both short nanosecond duration pulses to effectively treat pigmented lesions, and long millisecond duration pulses to effectively treat vascular lesions.

Apparatus 500 consists of a free running alexandrite laser 504 which pumps via a fiber optics 506 a passively Q-switched by a Cr⁴+:YAG (Chromium-doped Yttrium Aluminum Garnet) Q-switch 508 Neodymium doped (Nd-doped) laser medium or material 512. Passive Q-switching of the Nd:YLF by Cr⁴+:YAG compared with active Q-switching reduces the size and complexity of the switching process. Fiber delivery of the 755nm alexandrite laser pump energy isolates the thermal lensing of the alexandrite pump laser 504 from the pumped Neodimium:YLF laser 512. Fiber 506 also acts as a beam mode scrambler to achieve an uniform "top-hat" energy distribution of the pump beam at the Neodymium:YLF laser rod, which supports the Neodymium:YLF laser 512 output top-hat energy distribution being stable and insensitive to pump laser beam variations.

Alexandrite lasers emitting a wavelength of 755nm, which is commonly used for hair removal. Alexandrite laser is used here to pump a rare earth doped crystal laser operating with output near 1 micron wavelength. The hair removal lasers are readily available and not expensive. The laser host medium or material 512 can be for example, such as YAG, YSGG, YALO, CaWO4, or YLF. The laser medium dopants could be one of a group of the rare earth materials such as Pr, Nd, or Yb. A Nd:YLF (yttrium lithium fluoride) in particular was selected and tested. The output laser beam 514 of the Nd-doped laser medium or material 512 could be near 1 micron wavelength and in particular 1048nm when the host is YLF. The 1048nm wavelength of laser beam 514 could be frequency-doubled by a frequency doubling crystal 516 such as for example LBO or KTP to yield the green 524nm wavelength output. The laser output 520 so generated could be coupled into an optical fiber 550 and delivered to the treated skin segment containing a combination of pigmented and vascular lesions. A harmonic separator 524 could be used to separate the green output 520 from the unconverted infrared light 528 with wavelength of 1048nm. The unconverted infrared light 528 could be directed and absorbed in a laser light beam dump 532 while the green laser light 520 with wavelength of 524nm could be delivered to the treated skin segment containing a combination of pigmented and vascular lesions. Laser light beam dump 532 could be designed to effectively dissipate the unconverted infrared energy without getting damaged or causing a rise in temperature of other apparatus 500 components. Passive and active cooling mechanisms can be used as need to remove heat from the laser light beam dump 532.

Other elements in FIG. 5 include a highly reflective mirror 536 and a partially reflective mirror 540 forming the laser resonator or cavity, a lens 544 configured to efficiently couple the pumping laser beam emitted by alexandrite laser 504 to Nd;YLF laser medium 512, an optical system 546, which could be a beam shaping telescopic system. Second harmonic generator (SHG) 516 could be a Lithium Triborate LiB₃O₅ (also LBO) nonlinear optical crystal. The LBO crystal was selected, since it allows even at room temperature non-critical phase-matching (NCPM) for 0.8-1.1 µm wavelength. LBO possesses a relatively large angular acceptance bandwidth, reduces the beam quality requirements and supports low sensitivity to angular misalignment for the Nd:YLF laser. In addition LBO has a large effective SHG coefficient, which is about three times that of KDP. Further improvement of the LBO operation could be achieved by placing the LBO crystal into an oven 548 designed to maintain constant temperature of the LBO crystal and which may allow the temperature to be actively controlled to maximize frequency-doubling efficiency.

Use ofNd:YLF laser medium provides a number of advantages that simplify apparatus 500. The output wavelength of Nd:YLF laser medium is 1048 nm, which when frequency doubled yields a 524nm wavelength. As it has been explained above, the blood absorption at the 524nm wavelength is similar to that of the wavelength of 585nm, higher than at the wavelength of 595nm, but lower than at the wavelength of either 532nm or 577nm. The wavelength of 524nm is therefore more suitable than the other wavelengths for treatment of blood vessels of 40-135 micron diameter. The 524nm wavelength yields treatment results better than currently used 532nm wavelength. Blood absorption of the 524nm wavelength is lower than at 532nm and the laser light penetrates deeper into larger blood vessels. Another advantage of using Nd:YLF laser medium is its lower coefficient of refractive index change with temperature (thermal lensing). This reduces beam quality degradation due to heating in the laser rod in long pulse operation, which facilitates maintaining good output beam quality needed for efficient frequency doubling. The choice of Nd:YLF over other laser materials also reduces thermal lensing due to the lower refraction index changes of the YLF with temperature. It also offers natural linear polarization allowing compact and simple resonator design compared with the commonly used Nd:YAG resonator designs.

In one example of the apparatus, apparatus 500 supports generation of a dual wavelength pulse train, with the first wavelength (524 nm) being in the 500nm-600nm region of the spectrum and the second wavelength (1048 nm) being in the near infrared region of the spectrum. The harmonic separator 524 could be removed from the apparatus 500 to allow the 500nm-600nm laser energy and the unconverted near infrared laser energy or light to be combined and delivered to the treated segment of skin together. In addition to the 500nm-600nm wavelength light for vascular lesions with smaller diameter vessels, infrared light is also often used for slightly larger veins treatment, typically 500 microns to 2.5 mm in diameter. By combining two wavelengths (500nm-600nm and near infrared) together, vascular lesions and veins can be treated in a single treatment pass. The two wavelengths could be supplied or delivered in different treatment sequences or simultaneously to achieve synergistic effect. The fluence range of 500nm-600nm wavelength could be 3 J/cm² to 30 J/cm² while the near infrared wavelength could be 10 J/cm² to 300 J/cm² depending on vascular lesion type. The relation between the 500nm-600nm wavelength to the near infrared wavelength could also be defined as fluence ratio and would be in the range from 1: 3 to 1: 10.

The infrared portion (1048nm) of the laser light (optical energy) could be used to heat up skin collagen and stimulate new collagen production in skin. Therefore apparatus 500 in addition to treating vascular and pigmented lesions could also support a skin rejuvenation procedure (such as "laser toning") in a single treatment pass. A large fluence range can be applied for the skin rejuvenation procedure (5-80J/cm²) at 1048nm or 1064nm depending on subject skin phototype and laser beam spot diameter. As such the apparatus could support a method of using this combined laser wavelength treatment for pigmented lesions, vascular lesions, as well as for collagen remodeling resulting in skin toning in a single pass.

Using an Alexandrite laser 504 that is already being widely used in dermatology to pump a laser medium 512 to treat vascular lesions has the significant advantage of lower cost for user. Also because alexandrite laser pumping is much more efficient than flash lamp pumping, thermal issues in the pumped laser are reduced. Pumping with an alexandrite laser rather than with conventional flash-lamps increases pumping efficiency from less than 3% to over 50%, thereby reducing thermal lensing in the Nd:YLF laser rod. This supports the use of the mechanically less robust Nd:YLF rod as the laser medium without causing thermal rupture of the laser rod. The use of YLF as the laser medium yields a more optimal output wavelength for treating vascular lesions. It also leads to better output beam quality and higher peak powers when Q-switched, which supports the efficient frequency doubling.

FIG. 6 is a plot the amplitude versus time of an exemplary laser pulse train prescribed herein for the treatment of vascular and pigmented lesions on a skin segment. As it has been explained supra the authors of the current disclosure discovered that using a sequence of macro pulses 600 of laser light provided by pump laser with individual macro pulse 604 having a duration selected to match the thermal relaxation time of the treated blood vessels and filling-in each individual macro pulse with a train 608 of laser energy sub-pulses with each sub-pulse having a duration of 5 nanoseconds to 200 nanoseconds provides better than other treatment techniques results for treatment of melanosomes. A Q-switch could be used to generate a train of sub-pulses to fill-in each individual macro pulse. The length of the macro or envelop pulse 604 could be 0.1miliseconds to 100 milliseconds and each envelop pulse could contain tens to hundreds of sub-pulses. The sub-pulses 608 could have a repetition rate of 10kHz - 1MHz and the macro pulses 604 could have a repetition rate of 1 to 10 Hz. It has been also discovered that the combination of a long macro pulse made-up of a train of multiple short sub-pulses within the macro pulse train is more effective in treating a segment of skin with a combination of pigmented and vascular lesions. The fluence range of the treatment of the combination of pigmented and vascular lesions, could be from 3J/cm² to 30J/cm² depending on subject skin phototype, laser spot diameter, and duration of the pulse train.

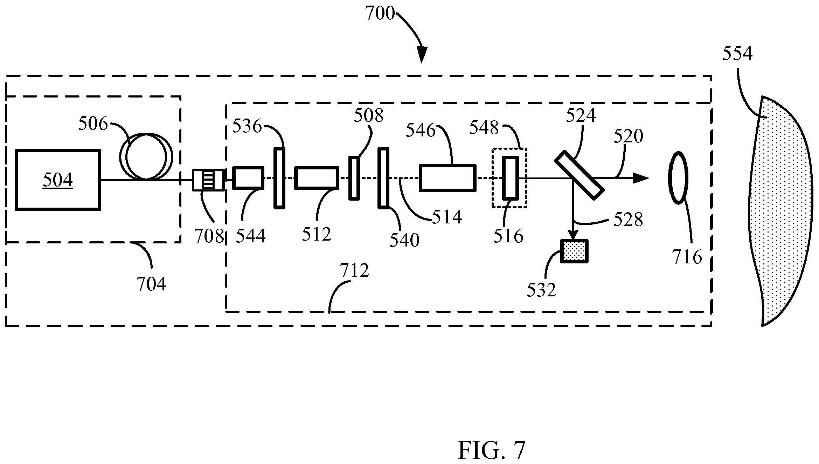

Fig 7 is another example of an apparatus for treatment of pigmented and vascular lesions on a skin segment. Apparatus 700 includes a separate packaging 704. Located in the packaging 704 is a pumping Alexandrite laser 504 coupled to fiber 506. The protruding from packaging 704 end of fiber 506 could be terminated by a SMA or similar fiber optics connector 708. The Nd:YLF laser 512, the frequency doubling crystal 516 and other components of apparatus 500 are relatively small in the size and could reside in a detachable handpiece 712. Handpiece 712 could be connected to packaging 704 with Alexandrite laser 504 with the help of connector 708. The distal end of handpiece 712 could be terminated by an aperture or by a lens 716 to relay the laser light to the treated skin segment 554.

Apparatuses 500 and 700 eliminate or reduce a number of problems existing with the current prevailing in the industry approaches:
- Pumping with an alexandrite laser rather than with conventional flash-lamps increases pumping efficiency from less than 3% to over 50%, thereby reducing thermal lensing in the Nd:YLF laser rod.
- The choice of Nd:YLF over other laser materials further reduces thermal lensing due to the lower refraction index changes of the YLF with temperature. It also offers natural linear polarization allowing compact and simple resonator design compared with the commonly used Nd:YAG resonator designs.
- The alexandrite pump is double-passed through the Nd:YLF, which reduces the required physical length of the Nd:YLF rod for complete absorption of pump light.
- Passive Q-switching of the Nd:YLF by Cr⁴⁺:YAG compared with active Q-switching reduces the size and complexity of the switching process.
- Fiber delivery of the 755nm alexandrite laser pump energy:
   ∘ Isolates the thermal lensing of the alexandrite pump laser from the pumped Nd:YLF laser.
   ∘ The fiber acts as a beam mode scrambler and achieves a top-hat distribution of the pump energy, which supports the Nd:YLF laser output top-hat energy distribution being stable and insensitive to pump laser beam variations.

It will be appreciated by persons skilled in the art that the present apparatus and method are not limited to what has been particularly shown and described hereinabove.

## Claims

1. An apparatus for laser treatment of pigmented lesions and vascular lesions, operable to generate a train of sub-pulses, the apparatus comprising:
a flash-lamp pumped free-running alexandrite laser (504) configured to generate a sequence of laser macro pulses (600);
an Neodymium doped laser medium (512) pumped via an optical fiber (506) by the free running alexandrite laser (504);
a passive Q-switch (508) configured to generate a train of sub-pulses;
a beam shaping telescope (546);
an optical fiber (550) for delivery to a segment of skin; and
a second harmonic generator (516) and a lens to focus laser light generated by the Neodymium doped laser medium (512) into said optical fiber (550);
**characterized in that**
the alexandrite laser is configured to generate the sequence of laser macro pulses with each laser macro pulse being selected to match the thermal relaxation time of blood vessels in the segment of skin and having a duration of 0.1 to 100ms; and
the passive Q-switch is configured to generate the train of sub-pulses with each train of sub-pulses being within an envelope of 0.1ms to 100ms and wherein a duration of the individual sub-pulses is in a range of 5ns to 200ns.

2. The apparatus according to claim 1 wherein the Neodymium doped laser medium (512) is a Nd:YLF rod.

3. The apparatus according to claim 1 or claim 2, wherein the passive Q-switch (508) is a Cr⁴⁺:YAG crystal.

4. The apparatus according to any preceding claim, wherein the second harmonic generator (516) is a Lithium Triborate (LiB3O5 or LBO) nonlinear optical crystal.

5. The apparatus according to any preceding claim, wherein the second harmonic generator (516) is a non-critically phase matched Lithium Triborate (LiB3O5 or LBO) nonlinear optical crystal.

6. The apparatus according to any preceding claim, wherein the second harmonic generator (516) is non-critically phase matched to support low sensitivity to angular misalignment.

7. The apparatus according to any preceding claim, wherein the second harmonic generator (516) produces a laser light with wavelength of 524nm.

8. The apparatus according to any preceding claim, wherein the optical fiber acts as a beam mode scrambler to achieve a top-hat distribution of the pump energy to support the Nd:YLF laser output top-hat energy distribution being stable and insensitive to pump laser beam variations.

9. The apparatus according to any preceding claim, wherein the train of laser light sub-pulses (608) is directable to a segment of skin and comprises laser light with a combination of two wavelengths of 524nm and 1048nm.

10. The apparatus according to claim 7 wherein the fluence ratio of a 524nm wavelength to a wavelength of 1048 nm is between 1 to 3 and 1 to 10.

11. The apparatus according to any preceding claim, wherein laser light fluence of the train of laser sub-pulses is between 3J/cm² and 30J/cm².

12. The apparatus according to claim 2, wherein the Nd:YLF laser provides natural linear polarization.

13. A non-therapeutic and non-surgical method of operating a laser light delivery apparatus operable to deliver laser light with a wavelength between 500nm and 600nm, comprising:
employing apparatus according to any one of claims 1-12 and delivering the laser light in a train (608) of sub-pulses (512) filling-in each laser macro pulse in a sequence of laser macro pulses, wherein the duration of the sub-pulses is in a range of 5ns to 200ns, and the duration of the macro pulses is 0.1ms to 100ms.

14. The method according to claim 13, wherein the laser light delivery apparatus delivers light energy as a combination of 500nm-600nm laser energy with near infrared laser energy.

## Patentansprüche

1. Vorrichtung zur Laserbehandlung von pigmentierten Läsionen und Gefäßläsionen, die funktionsfähig ist, einen Zug von Teilpulsen zu erzeugen, wobei die Vorrichtung umfasst:
einen mit Blitzlampen gepumpten, freilaufenden Alexandrit-Laser (504), der konfiguriert ist, eine Sequenz von Laser-Makropulsen (600) zu erzeugen;
ein Neodym-dotiertes Lasermedium (512), das über eine Glasfaser (506) durch den freilaufenden Alexandrit-Laser (504) gepumpt wird;
eine passive Güteschaltung (508), die konfiguriert ist, eine Folge von Teilpulsen zu erzeugen;
ein Strahlformungsteleskop (546);
eine Glasfaser (550) zur Abgabe an ein Hautsegment; und
einen Frequenzverdoppler (516) und eine Linse, um das vom Neodymdotierten Lasermedium (512) erzeugte Laserlicht in die optische Faser (550) zu fokussieren,
**dadurch gekennzeichnet, dass**
der Alexandrit-Laser konfiguriert ist, die Sequenz von Laser-Makropulsen zu erzeugen, wobei jeder Laser-Makropuls ausgewählt ist, sich an die thermische Relaxationszeit von Blutgefäßen im Hautsegment anzupassen und eine Dauer von 0,1 bis 100 ms aufzuweisen; und
die passive Güteschaltung konfiguriert ist, die Folge von Teilpulsen zu erzeugen, wobei sich jede Folge von Teilpulsen innerhalb einer Einhüllenden von 0,1 ms bis 100 ms befindet, und wobei eine Dauer der einzelnen Teilpulse in einem Bereich von 5 ns bis 200 ns liegt.

2. Vorrichtung nach Anspruch 1, wobei das Neodym-dotierte Lasermedium (512) ein Nd:YLF-Stab ist.

3. Vorrichtung nach Anspruch 1 oder Anspruch 2, wobei die passive Güteschaltung (508) ein Cr⁴⁺:YAG-Kristall ist.

4. Vorrichtung nach einem beliebigen voranstehenden Anspruch, wobei der Frequenzverdoppler (516) ein Lithiumtriborat (LiB3O5 oder LBO) nichtlinearer optischer Kristall ist.

5. Vorrichtung nach einem beliebigen voranstehenden Anspruch, wobei der Frequenzverdoppler (516) ein unkritisch phasenangepasster Lithiumtriborat (LiB3O5 oder LBO) nichtlinearer optischer Kristall ist.

6. Vorrichtung nach einem beliebigen voranstehenden Anspruch, wobei der Frequenzverdoppler (516) unkritisch phasenangepasst ist, um eine geringe Empfindlichkeit gegenüber Winkelfehlanpassungen zu unterstützen.

7. Vorrichtung nach einem beliebigen voranstehenden Anspruch, wobei der Frequenzverdoppler (516) ein Laserlicht mit einer Wellenlänge von 524 nm erzeugt.

8. Vorrichtung nach einem beliebigen voranstehenden Anspruch, wobei die Glasfaser als ein Strahlmodenmischer fungiert, um eine Top-Hat-Verteilung der Pumpenergie zu erreichen, um zu unterstützen, dass die Nd:YLF-Laserausgabe Top-Hat-Energieverteilung stabil und unempfindlich gegenüber Pumplaserstrahlvariationen ist.

9. Vorrichtung nach einem beliebigen voranstehenden Anspruch, wobei der Zug von Laserlicht-Teilpulsen (608) auf ein Hautsegment richtbar ist, und Laserlicht mit einer Kombination aus zwei Wellenlängen von 524nm und 1048nm umfasst.

10. Vorrichtung nach Anspruch 7, wobei das Fluenzverhältnis einer 524 nm-Wellenlänge zu einer Wellenlänge von 1048 nm zwischen 1 bis 3 und 1 bis 10 liegt.

11. Vorrichtung nach einem beliebigen voranstehenden Anspruch, wobei die Laserlichtfluenz der Folge von Laser-Teilpulsen zwischen 3J/cm² und 30J/cm² liegt.

12. Vorrichtung nach Anspruch 2, wobei der Nd:YLF-Laser eine natürliche lineare Polarisation bereitstellt.

13. Nicht-therapeutisches und nicht-chirurgisches Verfahren zum Betreiben einer Laserlichtabgabevorrichtung, die funktionsfähig ist, Laserlicht mit einer Wellenlänge zwischen 500 nm und 600 nm abzugeben, umfassend:
Verwenden der Vorrichtung gemäß einem beliebigen der Ansprüche 1-12 und Abgeben des Laserlichts in einer Folge (608) von Teilpulsen (512), die jeden Lasermakropuls in einer Sequenz von Lasermakropulsen ausfüllen, wobei die Dauer der Teilpulse in einem Bereich von 5 ns bis 200 ns liegt, und die Dauer der Makropulse 0,1 ms bis 100 ms beträgt.

14. Verfahren nach Anspruch 13, wobei die Laserlichtabgabevorrichtung Lichtenergie als eine Kombination von 500nm-600nm Laserenergie mit Nahinfrarotlaserenergie abgibt.

## Revendications

1. Appareil de traitement au laser de lésions pigmentées et de lésions vasculaires, utilisable pour produire un train de sous-impulsions, l'appareil comprenant :
un laser alexandrite relaxé à pompage par lampe flash (504) configuré pour produire une séquence de macro-impulsions laser (600) ;
un milieu laser dopé au néodyme (512) pompé par l'intermédiaire d'une fibre optique (506) par le laser alexandrite relaxé (504) ;
un commutateur-Q passif (508) configuré pour produire un train de sous-impulsions ;
un système télescopique de modelage de faisceau (546) ;
une fibre optique (550) pour le transport vers un segment de peau ; et
un générateur de seconde harmonique (516) et une lentille de focalisation de la lumière laser produite par le milieu laser dopé au néodyme (512) dans ladite fibre optique (550) ;
**caractérisé en ce que**
le laser alexandrite est configuré pour produire la séquence de macro-impulsions laser dont chaque macro-impulsion laser est sélectionnée pour correspondre au temps de relaxation thermique des vaisseaux sanguins dans le segment de peau et présente une durée comprise entre 0,1 et 100 ms ; et
le commutateur-Q passif est configuré pour produire le train de sous-impulsions, dont chaque train de sous-impulsions s'inscrit dans une enveloppe allant de 0,1 ms à 100 ms et dans lequel la durée des sous-impulsions individuelles est comprise entre 5 ns et 200 ns.

2. Appareil selon la revendication 1, dans lequel le milieu laser dopé au néodyme (512) est un barreau de Nd:YLF.

3. Appareil selon la revendication 1 ou la revendication 2, dans lequel le commutateur-Q passif (508) est un cristal de Cr⁴⁺:YAG.

4. Appareil selon l'une quelconque des revendications précédentes, dans lequel le générateur de seconde harmonique (516) est un cristal optique non linéaire de triborate de lithium (LiB3O5 ou LBO).

5. Appareil selon l'une quelconque des revendications précédentes, dans lequel le générateur de seconde harmonique (516) est un cristal optique non linéaire de triborate de lithium (LiB3O5 ou LBO) à accord de phase non critique.

6. Appareil selon l'une quelconque des revendications précédentes, dans lequel le générateur de seconde harmonique (516) est à accord de phase non critique pour permettre une faible sensibilité à un défaut d'alignement angulaire.

7. Appareil selon l'une quelconque des revendications précédentes, dans lequel le générateur de seconde harmonique (516) produit une lumière laser à une longueur d'onde de 524 nm.

8. Appareil selon l'une quelconque des revendications précédentes, dans lequel la fibre optique joue le rôle de brouilleur de mode de faisceau pour obtenir une distribution top-hat (en chapeau haut-de-forme) de l'énergie de pompage afin d'assurer la stabilité de la distribution d'énergie top-hat de sortie du laser Nd:YLF et son insensibilité aux variations de faisceau laser de pompage.

9. Appareil selon l'une quelconque des revendications précédentes, dans lequel le train de sous-impulsions de lumière laser (608) est orientable vers un segment de peau et comprend une lumière laser présentant une combinaison de deux longueurs d'onde, de 524 nm et 1048 nm.

10. Appareil selon la revendication 7, dans lequel le taux de fluence d'une longueur d'onde de 524 nm par rapport à une longueur d'onde de 1048 nm est compris entre 1 : 3 et 1 : 10.

11. Appareil selon l'une quelconque des revendications précédentes, dans lequel la fluence de la lumière laser du train de sous-impulsions laser est comprise entre 3 J/cm² et 30 J/cm².

12. Appareil selon la revendication 2, dans lequel le laser Nd:YLF apporte une polarisation linéaire naturelle.

13. Procédé non thérapeutique et non chirurgical d'utilisation d'un appareil d'émission de lumière laser utilisable pour émettre une lumière laser de longueur d'onde comprise entre 500 nm et 600 nm, comprenant :
l'utilisation de l'appareil selon l'une quelconque des revendications 1 à 12 et l'émission de la lumière laser sous forme d'un train (608) de sous-impulsions (512) remplissant chaque macro-impulsion laser d'une séquence de macro-impulsions laser, dans lequel la durée des sous-impulsions est comprise entre 5 ns et 200 ns et la durée des macro-impulsions est comprise entre 0,1 ms et 100 ms.

14. Procédé selon la revendication 13, dans lequel l'appareil d'émission de lumière laser émet de l'énergie lumineuse sous forme de combinaison d'énergie laser de 500 nm à 600 nm et d'énergie laser proche infrarouge.
